Europäisches Patentamt

**European Patent Office**

**Office européen des brevets**

(19)

(11) Veröffentlichungsnummer: **0 032 974**
A1

(12) **EUROPÄISCHE PATENTANMELDUNG**

(21) Anmeldenummer: **80106054.2**

(22) Anmeldetag: **06.10.80**

(51) Int. Cl.³: **C 07 C 39/07**
C 07 C 37/16, B 01 J 21/10
B 01 J 21/14, B 01 J 21/18

(30) Priorität: **28.01.80 US 116056**

(43) Veröffentlichungstag der Anmeldung:
**05.08.81 Patentblatt 81/31**

(84) Benannte Vertragsstaaten:
**BE CH DE FR GB LI**

(71) Anmelder: **CONOCO INC.**
**1000 South Pine Street P.O. Box 1267**
**Ponca City Oklahoma 74601(US)**

(72) Erfinder: **Leach, Bruce Eugene**
**1628 Dover**
**Ponca City, Oklahoma 74601(US)**

(74) Vertreter: **Patentanwälte Dipl.-Ing. A. Grünecker,**
**Dr.-Ing. H. Kinkeldey, Dr.-Ing. W. Stockmair,**
**Dr. rer. nat. K. Schumann, Dipl.-Ing. P. Jakob, Dr. rer.**
**nat. G. Bezold Maximilianstrasse 43**
**D-8000 München 22(DE)**

(54) **Verfahren zur Herstellung von 2,3,6-Trimethylphenol.**

(57) Meta-substituted phenolic materials are used in the production of 2,3,6-trimethylphenol by methylating the phenolic materials under specific conditions of temperature, pressure in the presence of a new catalyst in order to obtain 2,3,6-trimethylphenol in high selectivities. The new catalyst contains amorphous titanium components.

EP 0 032 974 A1

Croydon Printing Company Ltd.

A METHOD FOR THE SYNTHESIS OF 2,3,6-TRIMETHYLPHENOL

This invention relates to the vapor phase methylation of phenolic compounds. More specifically, this invention relates to the vapor phase methylation of meta-substituted phenolic compounds such as meta-cresol, 2,5-xylenol, 2,3-xylenol or mixtures of materials containing these isomers to produce 2,3,6-trimethylphenol.

2,3,6-Trimethylphenol is useful as an intermediate in Vitamin E synthesis. Until direct methylation methods were developed, 2,3,6-trimethylphenol having required purity for Vitamin E synthesis was expensive and difficult to obtain.

Both vapor and liquid phase methylation of phenolics are well known. Examples of such methylation techniques can be found in German Patent 1,817,243. Methylation of 2,6-xylenol to product 2,3,6-trimethylphenol was described in British Patent 1,254,660. U.S. Patent 3,979,464 also describes a method for methylating 2,6-xylenol to produce 2,3,6-trimethylphenol. However, all known methods suffer from some disadvantages. For example, when methylation is carried out in vapor phase reactions, useful catalyst life is extremely limited and numerous undesirable by-products are formed along with desired 2,3,6-trimethylphenol.

Liquid phase methylation allows the use of lower reaction temperatures, provides longer catalyst life and fewer undesirable by-products than do vapor phase reactions. However, liquid phase catalyst life, while superior to vapor phase catalyst life, requires excessively long residence times and excessively high pressures.

It is therefore an object of the present invention to provide an improved process for the vapor phase methylation of meta-substituted cresols or xylenols obtaining high reaction rates, acceptable catalyst life, and a high selectivity of 2,3,6-trimethylphenol. Other objects will become apparent to those skilled in this art as the description proceeds.

In accordance with the present invention an improved process for selectively increasing the yield of 2,3,6-trimethylphenol in vapor phase methylation of phenolic compounds is provided. The process is an improvement over the heretofore known processes in several regards, due mainly to the presence of a novel catalyst comprising amorphous titanium in combination with magnesium oxide and sulfate ion.

Concisely, the improved process of the present invention comprises a method for the synthesis of 2,3,6-trimethylphenol comprising reacting meta-substituted phenolics with methanol in the presence of a catalyst at

a) temperatures of from about 400°C to about 500°C

b) liquid hourly space velocities (LHSV) of from about .1 to about 10;

c) pressures of from about atmospheric to about 10 atmospheres;

d) a methanol mole ratio of from about 1 to about 10; preferably carried out

e) in the presence of from about 1 to about 15% water by weight based on the total weight of the feedstream; wherein the catalyst is an amorphous titanium/sulfate/ magnesium oxide catalyst.

In carrying out the process of the instant invention from about .1 to about 10 moles of methanol are used per mole of phenolic feed components, but from about 2 to about 8 mole is preferred and from about 3 to about 6 mole is most preferred. While prior art practices have required essentially pure alkylated phenol feedstocks such as 2,6-xylenol in order to carry out the methylation in a practical manner, the present invention can be carried out using meta-substituted phenolics. However, other phenolics can be present. Ideally, the mixture will contain fairly large amounts of meta-cresol, 2,5-xylenol and 2,3-xylenol (methyl substituents), although the process is operable when other substituted phenols are present. Very acceptable results can be obtained with these mixtures of phenolics.

The process of the present invention can be carried out efficiently in a batch reactor or a continuous flow reactor. The continuous flow reactor is preferred.

In the continuous flow reactor the residence time in the reactor is from about 10 to about 60 seconds.

Reaction conditions are temperatures of from about 400°C to about 500°C but temperatures of from about 420°C to about 480°C are preferred. Residence time of from about 5 to about 45 seconds are likewise preferred. During the course of the reaction, pressures of from about atmospheric to about 10 atmospheres are normally used, but a pressure of from about atmospheric to about 5 atmospheres is preferred and a pressure range of from about atmospheric to about 3 atmospheres is most preferred. An LHSV of from about .1 to about 10 can be used but an LHSV of from about .5 to about 5 is preferred and an LHSV of from about .5 to about 2 is most preferred. Methanol/feed mixture mole ratios of from about 1 to about 10 are normally used, but from about 2 to about 6 is preferred.

The presence of water in the feed mixture is not absolutely essential to the operation of the instant invention in the presence of the catalyst described herein. However, it has been found that the presence of appreciable amounts of water in the feedstream tend to maintain activity of the catalyst and increase the useful catalyst life before inactivation due to deposition of the phenolic feed material upon the catalyst. Normally, the feed mixture will contain from about 1 to about 15% by weight of water, but normally from about 5 to about 11% by weight will be used in order to adequately extend the catalyst life.

In the present invention, pressure controls residence time and temperature affects reaction rates. Therefore, pressure and temperature are balanced within the ranges given for optimum reaction of any given feedstream.

The catalyst of the present invention has been discovered to selectively yield high quantities of 2,3,6-trimethylphenol while reducing the amount of by-products formed. Such by-products, of course, must be removed before the requisite purity for Vitamin E synthesis can be achieved. The catalyst of the present invention is best described as an amorphous titanium/sulfate/magnesium oxide catalyst. The magnesium oxide provides the bulk of the catalyst, normally comprising from about 85 to about 98% by weight of the total catalyst weight. The amorphous titanium component of the catalyst can range from about 2 to about 10% by weight of the final catalyst and the sulfate component of the catalyst ranges from about 2 to about 10% by weight based upon the total catalyst component.

It has been found useful to add a minor amount of silica to the catalyst. Silica, while not essential to the process of the instant invention, is present in amounts of from about 1 to about 5% by weight based upon the weight of the finished catalyst.

In addition, in pelletizing operations for the catalyst of the present invention, it may be desirable to add from 1 to 2% by weight, based upon the weight of the finished catalyst, of graphite as a lubricating agent. The presence or absence of the graphite appears to have little or no effect upon the end products produced using the catalyst.

In the process of the present invention, mixtures of phenolic compounds are alkylated under the conditions set forth using the catalyst described. A large portion of 2,3,6-trimethylphenol will be recovered in the reaction product and can be removed easily by fractional distillation.

Selectivities to 2,3,6-trimethylphenol of over 80% can be obtained using feedstreams of pure material such as 2,3-xylenol and 2,5-xylenol. Selectivity is defined as the total production of the desired product (also called productivity) and is obtained by multiplying the total conversion of feedstock by the percent of desired product in total phenolics converted. Unconverted phenolics can be recycled as a feedstock along with by-products. Example 7 shows two passes through the reactor.

The invention is more concretely described with reference to the examples below wherein all parts and percentages are by weight unless otherwise specified. The examples are provided to illustrate the instant invention and not to limit it.

Reaction data disclosed herein were generated using a half inch stainless steel reactor containing 1/16 diameter catalyst extrudate. Flow in the reactor could be varied either upward or by gravity. The reactor contained approximately 15 cubic centimeters of catalyst. The reactor was heated using an electric furnace. Temperature was measured in the center of the reactor by a thermocouple. No cooling was provided. Temperatures given in the examples are the maximum temperatures of the reaction stream in the catalyst zone. After leaving the reactor, the product stream was condensed and the product distribution was determined using gas liquid chromatography (glc). Actual percentages were measured using a computerized program which measured the area under the glc curve. In all examples, 35-45 pounds per square inch gauge (psig) pressure was used.

### Example 1

The catalyst composition used in generating the examples of the present invention was prepared by blending Merck Maglite D magnesium oxide with ammonium sulfate, silica gel, graphite, and titanium isopropoxide diluted with isopropanol. The approximate composition of the catalyst, based upon the total weight of the finished catalyst, was magnesium oxide 87 weight percent, titanium oxide 5 weight percent, silica gel 3 weight percent, graphite 2 weight percent, and ammonium sulfate 3 weight percent.

The catalyst mixture was tableted and calcined 2 hours at 1000°F. The catalyst was used in various alkylation reactions for 568 hours, then recovered. In the following reactions, the same catalyst was used sequentially. This is possible because of the excellent catalyst life obtained.

Several reactions were carried out at 45 pounds per square inch gauge (psig) 1.0 to 1.4 LSHV, and 468°C. The catalyst charge used was 15 cubic centimeters.

### Example 2

A feed of 2,3-xylenol containing 4 moles of methanol per mole of 2,3-xylenol and 9 weight percent water was used.

The cresylic acid fraction after reaction at 1.0 LHSV is set forth in Table 1.

### Table 1

| Component | Wt. Percent |
| --- | --- |
| phenol | 0.09 |
| o-cresol | 0.72 |
| 2,6-xylenol | 2.69 |
| 2,3-dimethylanisole (DMA) | 1.19 |
| 2,3-xylenol | 2.64 |
| 2,3,6-trimethylanisole | 0.89 |
| 2,3,6-trimethylphenol (TMP) | 88.41 |
| other trimethylphenols | 0.67 |
| tetramethylphenols | 2.70 |

An extremely large percentage of 2,3,6-trimethylphenol was obtained.

### Example 3

A feed mixture of 2,5-xylenol containing 4 moles of methanol per mole of 2,5-xylenol in 9 weight percent water was used. The cresylic acid fraction after reaction at 1.4 LHSV is set forth in Table 2.

### Table 2

| Component | Wt. Percent |
| --- | --- |
| 2,6-xylenol | 0.15 |
| 2,5-dimethylanisole | 1.64 |
| 2,5-xylenol | 10.66 |
| 2,3-xylenol | 0.21 |
| 2,3,6-trimethylanisole | 1.55 |
| 2,3,6-trimethylphenol | 82.75 |
| other trimethylphenols | 0.77 |
| tetramethylphenols | 2.27 |

Example 4

Meta-cresol was used as a feed phenolic with a methanol/meta-cresol ratio of 5 to 1 respectively. Water (9%) was present in the feed. The results of the reaction under standard conditions are set forth in Table 3.

Table 3

| Component | Wt. Percent |
|---|---|
| phenol | 0.49 |
| m-methylanisole | 0.26 |
| m-cresol | 2.63 |
| 2,5-dimethylanisole | 0.91 |
| 2,5-xylenol | 30.58 |
| 2,3-xylenol | 6.70 |
| 2,3,6-trimethylanisole | 1.54 |
| 2,3,6-trimethylphenol | 54.07 |
| other trimethylphenols | 1.11 |
| tetramethylphenols | 1.71 |

Example 5

A stream of mixed phenolics containing approximately 90% by weight of 2,5-xylenol was used in a reaction. The crude phenolic stream is referred to as "cresylic acid streams" since it is a mixture of alkylated phenols. Feed to the reactor consisted of a 3 to 1 mole ratio of methanol to the cresylic acid, with 9% water added by weight. The reaction was carried out at 1.4 LHSV at 368°C. The feed analysis and product analysis of the reaction are set forth in Table 4.

Table 4

| Component | Feed | Wt.<br>Percent |
|---|---|---|
| 2,5-dimethylanisole | - | 2.34 |
| 2,6 Xylenol | 0.22 | 0.20 |
| 2,4-/2,5-xylenol | 93.36 | 9.95 |
| 2,3-xylenol | 0.68 | Tr |
| 2,4,6-trimethylphenol | 0.72 | 10.45 |
| 2,3,6-trimethylphenol | 2.16 | 72.50 |
| other trimethylphenols | 0.93 | 0.35 |
| pentamethylbenzene | 1.97 | 1.92 |
| tetramethylphenols | - | 2.30 |

Example 6

The crude stream containing 2,3 and 2,5-xylenol as major components together with mixtures in minor amounts of other alkylated phenols were used as a feedstream. A mole ratio of 3 to 1 methanol cresylic acid and 9 weight percent water was used. The reaction was carried out at 1.4 LHSV and 468°C. The results of the reaction are set forth in Table 5.

Table 5

| Component | Feed | Product |
|---|---|---|
| o-cresol | 0.63 | 0.09 |
| m,p-cresol | 0.27 | 0.13 |
| 2,6-xylenol | 9.38 | 9.94 |
| 2,4-/2,5-xylenol | 40.78 | 1.19 |
| 2,3-xylenol | 18.23 | 0.68 |
| 2,4,6-trimethylphenol | 25.79 | 44.83 |
| 2,3,6-trimethylphenol | 3.08 | 37.53 |
| other trimethylphenols | 0.12 | 0.24 |
| pentamethylbenzene | 1.73 | 1.82 |
| tetramethylphenols | - | 3.54 |

## Example 7

Use of m-p-cresol mixture as feedstock for preparation of 2,3,6-trimethylphenol was tested. m-p-Cresol 108g was mixed with 192g of methanol to give a feedstock having a mole ratio methanol/phenolic of 6/1. The m-/p ratio was 2.9. Reaction conditions were 470°C, 1 LHSV and 50 psig. Water was present in the feed (9 wt %). The entire product stream of the first reaction was used as the feed stream for the second reaction.

### Table 6

| Component | First Pass Through Reactor | Second Pass Through Reactor |
|---|---|---|
| anisoles | 0.06 | 0.37 |
| o-cresol | 3.28 | 2.28 |
| 2,6-DMA | 0.37 | 0.09 |
| m,p-cresol | 2.83 | 0.27 |
| 2,6-xylenol | 4.20 | 5.89 |
| 2,5-xylenol | 27.04 | 9.33 |
| 2,3-xylenol | 5.14 | 1.97 |
| 2,4,6-TMP | 16.78 | 22.72 |
| 2,3,6-TMP | 32.27 | 46.18 |
| other TMP | 3.80 | 2.44 |
| Tetramethylphenols | 4.23 | 8.46 |

## Example 8

As a comparative example to the preferred catalyst containing a minor amount of silica, a reaction was carried out wherein the catalyst contained no silica. The feed and reaction conditions of Example 4 were duplicated. The silica-free catalyst was prepared by grinding 5.4 g $MgSO_4$ and adding to 77.8 g MgO powder (Merck Maglite D). Titanium isopropoxide (6.5 g) was diluted with an equal weight of isopropanol, and the resulting mixture added to the powder. The liquid and powder were well mixed. Water (50 cc) was added to the mixture, which was then dried, sifted through a 60 standard mesh screen, and tableted. The tablets were dried overnight at 150°C, then calcined 2 hours at 500°C prior to use. The tablets were more easily crushed than similar tablets formed which contained silica.

The results of the reaction carried out using this catalyst are set forth in Table 7.

Table 7

| Component | Product |
| --- | --- |
| phenol | Tr |
| m-anisole | Tr |
| m-cresol | Tr |
| 2,5-dimethylanisole | 1.78 |
| 2,4/2,5-xylenol | 9.75 |
| 2,3-xylenol | 0.42 |
| 2,4,6-trimethylphenol | 11.10 |
| 2,3,6-trimethylphenol | 66.89 |
| other trimethylphenols | 0.64 |
| pentamethylbenzene | 1.62 |
| tetramethylphenols | 7.61 |

It will be apparent that the improved process provided herein is much superior to those provided by the prior art. Catalyst life is extended, selectivities are greatly increased, and impure feedstocks can be used while using reaction conditions of low pressure and temperatures no higher than those taught necessary by the prior art.

While certain embodiments and details have been shown for the purpose of illustrating this invention, it will be apparent to those skilled in this art that various changes and modifications may be made herein without departing from the spirit or scope of the invention.

I claim:

1.   A method for the synthesis of 2,3,6-trimethylphenol comprising

    a)   contacting meta-substituted phenolic materials with methanol in a vapor phase,

    b)   at a temperature of from about 400°C to about 500°C;

    c)   at a pressure of from about atmospheric to about 10 atmospheres, and

    d)   at a methanol/phenolic mole ratio of from about 1 to about 10;

wherein the catalyst is a magnesium oxide catalyst promoted with amorphous titanium and sulfate ions.

2.   A method as described in claim 1 wherein the reaction is carried out in a continuous flow reactor.

3.   A method as described in claim 2 wherein the reaction is carried out at an LHSV of about .1 to about 10.

4.   A method as described in claim 3 wherein the feed mixture contains water in an amount of from about 1 to about 15 percent by weight.

5.   A method as described in claim 4 when the catalyst component ratio of from about 2 to about 10 weight percent amorphous titanium, about 85 to about 98% by weight of magnesium oxide, and from about 2 to about 10% by weight sulfate based on the total weight of the catalyst.

6.   A method as described in claim 5 wherein in addition from about 1 to about 5% by weight of silica is added based on the weight of the final catalyst.

7.   A method as described in claim 3 wherein in addition from about 1 to about 3% graphite is added based by weight upon the weight of the final catalyst.

8.    A method as described in claim 4 wherein the reaction is carried out at a temperature of from about 420 to about 480°C, a liquid hourly space velocity of from about .5 to 2, and a pressure of from about atmospheric to about 5 atmospheres.

9.    A method as described in claim 8 wherein the feed mixture is primarily meta-cresol, 2,3-xylenol and 2,5-xylenol.

10.    A method as described in claim 9 wherein the materials other than 2,3,6 are recovered and reused as a feedstock.

European Patent Office

**EUROPEAN SEARCH REPORT**

| DOCUMENTS CONSIDERED TO BE RELEVANT | | | CLASSIFICATION OF THE APPLICATION (Int. Cl.³) |
|---|---|---|---|
| Category | Citation of document with indication, where appropriate, of relevant passages | Relevant to claim | |
| | US - A - 3 959 394 (AKIRA TASAKA)<br>+ Claims 1,10 +<br>-- | 1,2,8,9 | C 07 C 39/07<br>C 07 C 37/16<br>B 01 J 21/10<br>B 01 J 21/14<br>B 01 J 21/18 |
| | GB - A - 1 125 087 (COAL TAR RESEARCH)<br>+ Claims 1,8 +<br>-- | 1,2 | |
| | US - A - 3 971 832 (YOSHIHISA WATANABE)<br>+ Claims 1-3; example 6 +<br>-- | 1-3,8,9 | TECHNICAL FIELDS SEARCHED (Int. Cl.³) |
| | GB - A - 1 428 643 (F. HOFFMANN-LA ROCHE)<br>+ Claims 1,3,5,7; example 1 +<br>-- | 1,2,8,9 | C 07 C 39/00<br>C 07 C 37/00 |
| | GB - A - 1 451 091 (TEIJIN)<br>+ Claims 1,5,8,11 +<br>---- | 1-4,8 | |

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

| X | The present search report has been drawn up for all claims | | |
|---|---|---|---|
| Place of search<br>VIENNA | Date of completion of the search<br>24-03-1981 | Examiner<br>REIF | |

EPO Form 1503.1  06.78